# EUROPÄISCHE PATENTSCHRIFT

(11) **EP 1 530 738 B1**
(45) Veröffentlichungstag und Bekanntmachung des Hinweises auf die Patenterteilung: **17.05.2006**
(21) Anmeldenummer: 03792306.7
(22) Anmeldetag: 13.08.2003
(51) Int. Cl.: G02B 21/00, A61B 19/08

(54) **STERILER MIKROSKOP-BEZUG MIT OBJEKTIVADAPTER**
STERILE MICROSCOPE COATING COMPRISING AN OBJECTIVE ADAPTER
REVETEMENT DE MICROSCOPE STERILE COMPORTANT UN ADAPTATEUR D'OBJECTIF

(30) Priorität: 16.08.2002 DE 10238611; 23.10.2002 DE 10250811
(43) Veröffentlichungstag der Anmeldung: 18.05.2005
(73) Patentinhaber: Stuemed GmbH, 78532 Tuttlingen (DE)
(72) Erfinder: MÜLLER, Katharina, 78532 Tuttlingen (DE)
(74) Vertreter: Gleiss, Alf-Olav
(86) Internationale Anmeldenummer: PCT/EP2003/008964
(87) Internationale Veröffentlichungsnummer: WO 2004/019107

(56) Entgegenhaltungen:
- EP-A- 0 941 706
- US-A- 5 467 223
- US-A- 5 682 264
- US-A- 6 024 454

## Beschreibung

Die Erfindung betrifft ein Abdecksystem gemäß Oberbegriff des Anspruchs 1 sowie eine Vorrichtung zum sterilen Abdecken eines Mikroskops gemäß dem Oberbegriff des Anspruchs 18.

Abdecksysteme der hier angesprochenen Art sind bekannt (EP-A-0 941 706, US-A-5 467 223, US-A-5 682 264). Sie dienen dazu, einen vorzugsweise zylindrischen Gegenstand, insbesondere ein Linsensystem eines Mikroskops, abzudecken. Es sind Systeme bekannt, die einen mit einem optischen Fenster versehenen Ring aufweisen, der über das Linsensystem gestülpt werden kann. An dem Ring kann ein Folienmaterial befestigt sein, mit dessen Hilfe sich ganze Mikroskope, vorzugsweise Operationsmikroskope, ohne deren Funktion zu beeinträchtigen, steril abdecken lassen.

Ein weiteres derartiges Abdecksystem zum Befestigen an einem Linsensystem eines Mikroskops weist einen über ein Ende des Linsensystems stülpbaren Abdeckring mit mindestens einem Adapterring und mit mindestens zwei zylindermantelförmigen Innenflächen mit unterschiedlichen Durchmessern zum Adaptieren des Abdecksystems auf, wobei der mindestens eine Adapterring in den über das Endes des Linsensystems des Mikroskops stülpbaren Abdeckring einbringbar ist.

Nachteilig ist, dass die Ringe solcher Abdecksysteme insbesondere unter sterilen Bedingungen, beispielsweise mit Handschuhen, schwer handhabbar sind und möglicherweise herunterfallen. Dies kann auch bei einem unbeabsichtigten Trennen des Abdeckrings vom Adapterring geschehen, wobei ein zu Boden gefallener Ring kontaminiert und damit unbrauchbar wird.

Aufgabe der Erfindung ist es daher, ein Abdecksystem der oben genannten Art zu schaffen, dessen Handhabbarkeit so verbessert ist, dass die oben genannten Nachteile sich nicht einstellen.

### * (US-A 6,024,454) *

Zur Lösung dieser Aufgabe wird ein Abdecksystem vorgeschlagen, das die in Anspruch 1 genannten Merkmale aufweist. Dieses weist mindestens zwei, vorzugsweise im Wesentlichen zylindermantelförmige, Innenflächen mit unterschiedlichen Durchmessern zum Adaptieren des Abdecksystems auf. Damit ist das Abdecksystem an mindestens zwei verschiedene Ausführungen von zylindrischen Gegenständen, insbesondere Linsensystemen von Mikroskopen, mit verschiedenen Durchmessern adaptierbar. Dabei ist eine erste im Wesentlichen zylindermantelförmige Innenfläche an einen ersten Außendurchmesser eines Linsensystems eines Mikroskops und eine zweite im Wesentlichen zylindermantelförmige Innenfläche an einen zweiten Durchmesser eines Linsensystems eines Mikroskops angepasst Das Abdecksystem kann also wahlweise durch Anlegen einer der im Wesentlichen zylindermantelförmigen Innenflächen über das Ende verschieden großer zylindrischer Gegenstände gestülpt werden.

Die Anpassung des Abdecksystems an verschieden große Durchmesser erfolgt durch einen Abdeckring mit mindestens einem Adapterring, wobei sich das Abdecksystem dadurch auszeichnet, dass der mindestens eine Adapterring mit einer radialen nach außen stehenden und damit über die Außenfläche des Abdeckrings ragenden Grifflasche versehen ist. Damit ist der Adapterring auch mit Handschuhen besser erfassbar. Außerdem zeichnet sich das Abdecksystem dadurch aus, dass in die Oberfläche des Abdeckrings an die Anzahl der Grifflaschen angepasste Vertiefungen eingebracht sind, in die die mit dem mindestens einen Adapterring versehene Grifflasche im zusammengesetzten Zustand der Ringe des Abdecksystems eingesetzt werden. Dadurch kann die Grifflasche versenkt untergebracht und eine ebene Oberfläche der ineinander gesetzten Ringe des Abdecksystems realisiert werden. Schließlich zeichnet sich das Abdecksystem dadurch aus, dass der mindestens eine Abdeckring einen Anschlag, insbesondere eine Stufe für einen weiteren Adapterring aufweist und dass auf der Innen- und/oder Außenfläche des Adapterrings und/oder der Adapterringe Noppen, Rippen und/oder Vorsprünge vorgesehen sind. Diese verhindern ein unbeabsichtigtes Abfallen eines der Ringe, was zu einer Kontamination desselben führen würde.

Weitere Ausgestaltungen ergeben sich aus den übrigen Unteransprüchen.

Die der Erfindung zu Grunde liegende Aufgabe wird außerdem durch eine Vorrichtung zum sterilen Abdecken eines Mikroskops, insbesondere eines Operationsmikroskops, mit einer das Mikroskop einhüllenden Abdeckfolie gemäß Anspruch 18 gelöst. Diese Vorrichtung zeichnet sich durch ein Abdecksystem nach einem der Ansprüche 1 bis 17 aus.

Die Erfindung wird im Folgenden anhand der Zeichnung näher erläutert. Es zeigen:
- Figur 1: einen Teilschnitt einer nicht zur Erfindung gehörigen Vorrichtung zum sterilen Abdecken eines Mikroskops mit einem angedeuteten Linsensystem und
- Figuren 2 bis 5: Ausschnitte von Teilschnitten verschiedener Abdecksysteme mit unterschiedlich vielen Adapterringen, wobei nicht alle Abdecksysteme zur Erfindung gehören.

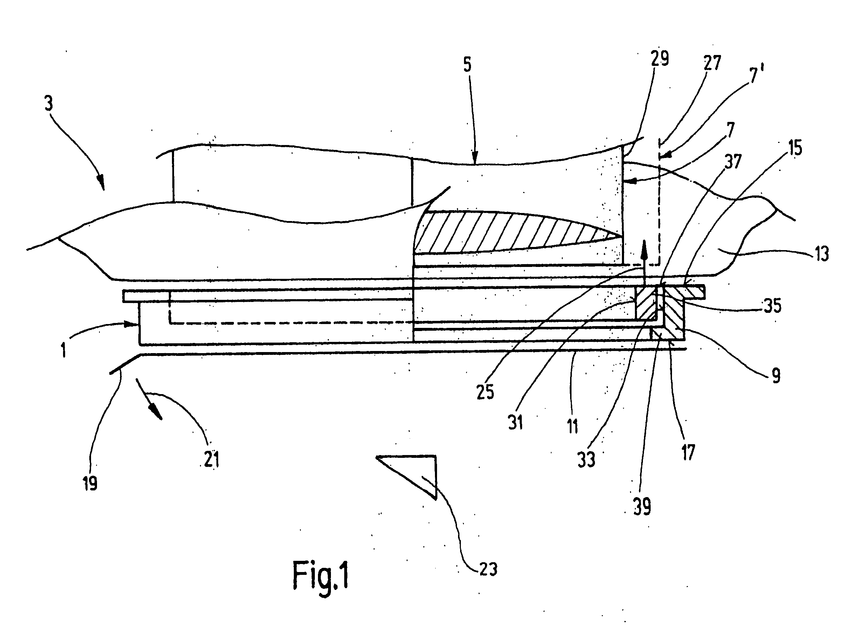

Figur 1 zeigt zur Erläuterung des technischen Gebiets einen Teilschnitt eines Abdecksystems 1, das nicht zur Erfindung gehört und das Teil einer Vorrichtung 3 zum sterilen Abdecken eines Mikroskops 5 ist. Von dem Mikroskop 5 ist lediglich ein Teil eines Linsensystems 7 dargestellt.

Das Abdecksystem 1 weist einen Abdeckring 9 auf, der mit einem Sichtfester 11 sowie einer Abdeckfolie 13 verbunden ist Hierzu weist der Abdeckring 9 eine obere Verbindungsfläche 15 sowie eine dieser gegenüberliegende untere Verbindungsfläche 17 auf. Die Abdeckfolie 13 und das Sichtfenster 11 sind mittels einer beliebigen Verbindungstechnik über die Verbindungsflächen 15, 17 mit dem Abdeckring 9 verbunden. Bevorzugt werden Verfahren wie Verkleben, Verschweißen oder Verschmelzen.

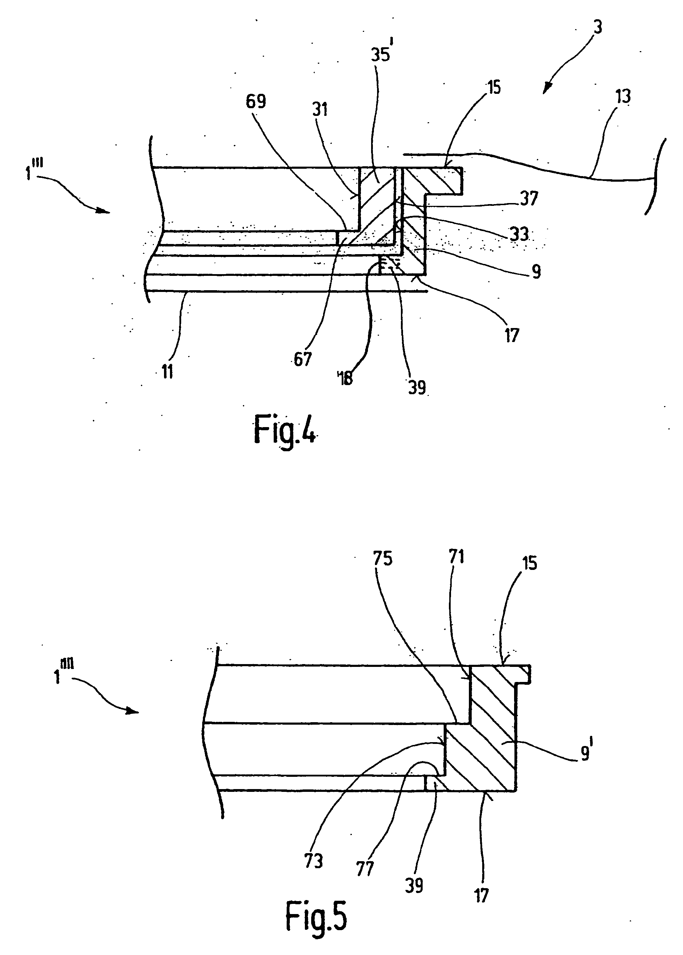

Bei einem hier nicht dargestellten Ausführungsbeispiel ist das Sichtfenster 11 mit dem Abdeckring 9 verklipst. Hierzu weist der Abdeckring 9 eine zu seiner Mittelachse hin geöffnete, umlaufende oder zumindest teilweise umlaufende Nut auf, in die das Sichtfenster 11, das an die Form der Nut angepasst ist, einklipsbar ist In Figur 4 ist durch Punkte 18 beispielhaft eine mögliche Positionierung an dem Abdeckring 9 sowie die mögliche Form einer solchen Nut angedeutet Es ist möglich, die umlaufende Nut des Abdeckrings 9 an einem kleinen Umfangsbereich so weit zu öffnen, dass diese in eine in Orientierung der Figur 1 nach unten geöffnete Ausnehmung übergeht, also in diesem Bereich keine Haltekräfte auf das Sichtfenster 11 übertragen werden können. Dies ermöglicht es, dass das Sichtfenster 11 in diesem Bereich zum Einbringen und Herausnehmen aus dem Abdeckring 9 gegriffen werden kann. Um das Greifen zu erleichtern, kann das Sichtfenster 11 eine der Ausnehmung des Abdeckrings 9 entsprechende, von der ansonsten runden Form abweichende Vergrößerung, insbesondere eine Lasche, aufweisen, die leicht ergriffen werden kann. Vorteilhaft ist, wenn für das Sichtfenster 11 und den Abdeckring 9 eine Materialkombination derart gewählt wird, dass der Abdeckring 9 elastisch verformbar ist, insbesondere aus einem gummiartigen Material besteht, und das Sichtfenster 11 so stabil ausgelegt ist, dass sich eine sichere Verbindung ergibt.

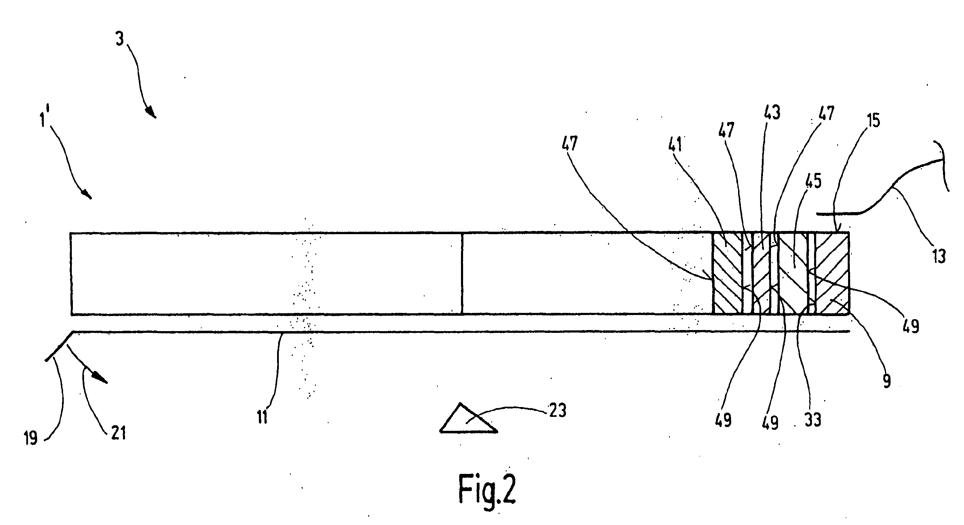

Besonders bevorzugt ist das Sichtfenster 11 mittels einer lösbaren Klebeverbindung an dem Abdeckring 9 befestigt Zum Lösen der Klebeverbindung weist das Sichtfenster 11, wie in Figur 2 dargestellt, eine Vorrichtung 19, die hier als greifbare Lasche ausgeführt ist, auf. Die Lasche des Sichtfensters 11 kann mit zwei Fingem gegriffen werden und in Richtung eines Pfeiles 21 gezogen werden, so dass sich das Sichtfenster 11 vom Abdeckring 9 löst Dies ist insbesondere dann sinnvoll, wenn das Sichtfenster 11 verschmutzt ist und/oder trotz bester optischer Eigenschaften des Sichtfensters 11 die Bildqualität nicht ausreicht und dieses entfernt werden muss.

Solange das Sichtfenster 11 mit dem Abdeckring 9 verbunden ist, trennt dieses gemeinsam mit der Abdeckfolie 13 das Mikroskop 5 von einem sterilen Bereich ab, in dem sich beispielsweise ein zu beobachtendes, steril zu haltendes Objekt 23 befindet Um das nur schwer zu sterilisierende Mikroskop 5 von dem steril zu haltenden Objekt 23 abzutrennen, wird das Abdecksystem 1 der Vorrichtung 3 in Richtung eines Pfeiles 25 über das Linsensystem 7 des Mikroskops 5 gestülpt und dort mittels eines Presssitzes fixiert. Anschließend kann die Folie 13 mittels Befestigungsmitteln ebenfalls am Mikroskop 5 fixiert werden. Insbesondere bei Operationen ist es besonders wichtig, dass sich das zu beobachtende Objekt 23 in einem sterilen Raum befindet, was sich durch Verpacken eines als Operationsmikroskop ausgebildeten Mikroskops 5 mit der Vorrichtung 3 mit dem Abdecksystem 1 gewährleisten lässt.

Das Abdecksystem 1 der Vorrichtung 3 ist an zwei verschiedene Außendurchmesser 27, 29 adaptierbar. Hierzu weist das Abdecksystem 1 zwei im Durchmesser unterschiedlich ausgelegte im Wesentlichen zylindermantelförmige Innenflächen 31, 33 auf. Dabei werden eine erste kleinere im Wesentlichen zylindermantelförmige Innenfläche 31 durch einen Adapterring 35 und eine im Durchmesser größere im Wesentlichen zylindermantelförmige Innenfläche 33 durch den Abdeckring 9 gebildet. In Figur 1 ist das Abdecksystem auf einen kleineren Außendurchmesser 29 des Linsensystems 7 adaptiert.

In diesem Zustand stehen eine Außenfläche 37 des Adapterrings 35 mit der im Wesentlichen zylindermantelförmigen Innenfläche 33 des Abdeckrings 9 sowie der Durchmesser 29 des Linsensystems 7 mit der im Wesentlichen zylindermantelförmigen Innenfläche 31 des Adapterrings 35 so miteinander in Anlagekontakt, dass je ein Presssitz zwischen dem Abdeckring 9 und dem Adapterring 35 sowie dem Adapterring 35 und dem Linsensystems 7 gebildet wird. Der Abdeckring 9 wird also über den Adapterring 35 über zwei Presssitze an dem Außendurchmesser 29 des Linsensystems 7 des Mikroskops 5 fixiert. Die Durchmesser der Innenfläche 31 des Adapterrings 35 sowie der Innenfläche 33 des Abdeckrings 9 und die Außenfläche 37 des Adapterrings 35 sind so aufeinander und auf den Durchmesser 29 des Linsensystems 7 abzustimmen, dass die sich durch den Reibschluss der Presssitze ergebenden Haltekräfte ausreichen, um das Abdecksystem 1 sicher an dem Linsensystem 7 befestigen zu können. Wenn für den Abdeckring 9 und/oder den Adapterring 35 relativ weiche elastische Materialien, insbesondere Gummi, gewählt werden, können entsprechend größere Differenzen der Durchmesser vorgesehen werden. Dies ist vorteilhaft, weil schon allein durch die Herstellung des Presssitzes an dem Linsensystem 7, ohne dass dadurch die Bedienkräfte zum Anbringen übermäßig ansteigen, eine gewisse Adaption erfolgt. In diesem Fall können also bereits durch die Presssitze Fertigungstoteranzen der einzelnen Mikroskophersteller ausgeglichen werden.

Zur Adaption des Abdecksystems 1 wird der Adaptening 35, bevorzugt mittels einer Entnahmehilfe, steril aus dem Abdeckring 9 entnommen. Sobald der Adapterring 35 entnommen ist, ist die Vorrichtung 3 des Abdecksystems 1 an einen größeren Außendurchmesser 27, der hier gestrichelt angedeutet ist, eines größeren Linsensystems 7' adaptiert. Die Fixierung der Vorrichtung 3 des Abdecksystems 1 am Linsensystem 7' erfolgt ebenfalls über einen Presssitz, der in diesem Fall zwischen dem Außendurchmesser 27 des Linsensystems 7' und der im Wesentlichen zylindermantelförmigen Innenfläche 33 des Abdeckrings 9 direkt herstellbar ist

Der Abdeckring 9 weist einen von seiner Innenfläche 33 nach innen ragenden Vorsprung 39 auf, der einen Anschlag für den Adapterring 34 bildet.

Figur 2 zeigt ein weiteres nicht zur Erfindung gehörendes Abdecksystem 1' mit Adapterringen 41, 43, 45. Gleiche Teile, die anhand von Figur 1 erläutert wurden, sind mit gleichen Bezugsziffern versehen, so dass insoweit auf die Beschreibung zu Figur 1 verwiesen wird.

Die Adapterringe 41, 43, 45 und der Abdeckring 9 weisen jeweils die gleiche Höhe auf und sind jeweils ineinander einbringbar. Jeder der Adapterringe 41, 43, 45 weist jeweils eine im Wesentlichen zylindermantelförmige Innenfläche 47 und eine im Wesentlichen zylindermantelförmige Außenfläche 49 auf. Die im Wesentlichen zylindermantelförmigen Innenflächen 47 sind auf verschiedene Außendurchmesser von Linsensystemen beziehungsweise abzudeckenden zylindrischen Gegenständen so abgestimmt, dass sie mit diesen mittels eines Presssitzes verbindbar sind. Die im Wesentlichen zylindermantelförmigen Außenflächen 49 der Adapterringe 41, 43, 45 sind jeweils so auf den nächst größeren Adapterring 43, 45 beziehungsweise den Abdeckring 9 abgestimmt, dass wahlweise eine beliebige Anzahl von Ringen ineinander mittels Presssitzen fixierbar sind.

Es wird deutlich, dass wahlweise durch Herausnehmen oder Einlegen eines oder mehrerer der Adapterringe 41, 43, 45 die Vorrichtung 3 an insgesamt vier verschiedene Außendurchmesser von zylindrischen Gegenständen adaptierbar ist

Vorteilhaft ist auch, dass sich die Adapterringe 41, 43, 45 auf der dem steril zu beobachtenden Objekt 23 gegenüberliegenden Seite des Sichtfensters 11, also im nicht sterilen Bereich befinden. Somit ist es auch denkbar, dass die Adapterringe 41, 43, 45 mit der bloßen Hand, also nicht steril entnommen werden.

Das Sichtfenster 11 ist aus einem hochtransparenten, einen störungsfreien Blick auf das Objekt 23 gewährenden Material gefertigt. In Frage kommen Glas, Plexiglas und/oder ähnliche Materialien oder diese aufweisende Verbundwerkstoffe. Besonders bevorzugt wird ein Sichtfenster 11 aus einer 300 µm starken Polycarbonatfolie, die geeignet ist, Reflexe, Spiegelungen, Ucht- beziehungsweise Farbbeeinträchtigungen zu vermeiden. Besonders bevorzugt werden Sichtfenster 11, die Cycloolefincopolymer, ein transparentes Copolymer auf der Basis von cyclischen und linearen Olefinen, umfassen, insbesondere aus einem Material bestehen, das zum Beispiel thermoplastisches Olefin-Polymer amorpher Struktur aufweist Dieses Material ist aufgrund seiner hohen Transparenz, seiner geringen Neigung zur Doppelbrechung, hohen Steifigkeit, Steritisierbarkeit und seiner hohen Wärmeformbeständigkeit besonders gut für das Sichtfenster 11 geeignet. Die Materialstärke ist in weiten Grenzen variierbar und beträgt bevorzugt 300 µm.

Somit wird ein durch das Abdecksystem 1, 1' abgedecktes Unsensystem 7, 7' eines Mikroskops 5 in keinster Weise in seiner Funktion beeinträchtigt.

Die in Figur 2 gezeigten Adapterringe 41, 43, 45 und der Abdeckring 9 sind aus einem weichen, anpassungsfähigen, vorzugsweise elastisch verformbaren Vollmaterial gefertigt Besonders bevorzugt kann dazu weiches Polyethylen verwendet werden.

Darüber hinaus ist es auch denkbar, beispielsweise den Abdeckring 9 und/oder einem oder alle Adapterringe 41,43,45, um Material einzusparen, innen hohl auszuführen. Anstelle des Vollmaterials kann also auch ein ringförmiges Profil verwendet werden, das eine Innen- und eine Außenwand aufweist. Die Wände sind in einem Abstand zueinander angeordnet, so dass sich -im Querschnitt gesehenquasi ein rechteckförmiges U-Profil ergibt. Dies ermöglicht eine materialsparende und kostengünstige Fertigung. Der Freiraum zwischen der Innen- und der Außenwand kann von -vorzugsweise radial verlaufenden- Verstärkungsrippen überspannt werden. Bei der Realisierung der Ringe ist darauf zu achten, dass noch ein genügend fester Presssitz mit dem nächstliegenden kleineren/größeren Ring und dem Linsensystem 7' des Mikroskops 5 sichergestellt ist.

Bei einem vorteilhaften Abdecksystem betragen die Durchmesser der im Wesentlichen zylindermantelförmigen Innen-/Außenflächen 47, 33, 49, der Adapterringe 41, 43, 45 beziehungsweise des Abdeckrings 9 -also die Grundmaße der Presssitze- zirka 48 mm, 65 mm, 70 mm, 87,5 mm. Diese Maße entsprechen den gängigen Außendurchmessern von Linsensystemen 7, 7'. Ein Anpassen an beliebige andere Maße/Linsensysteme ist möglich.

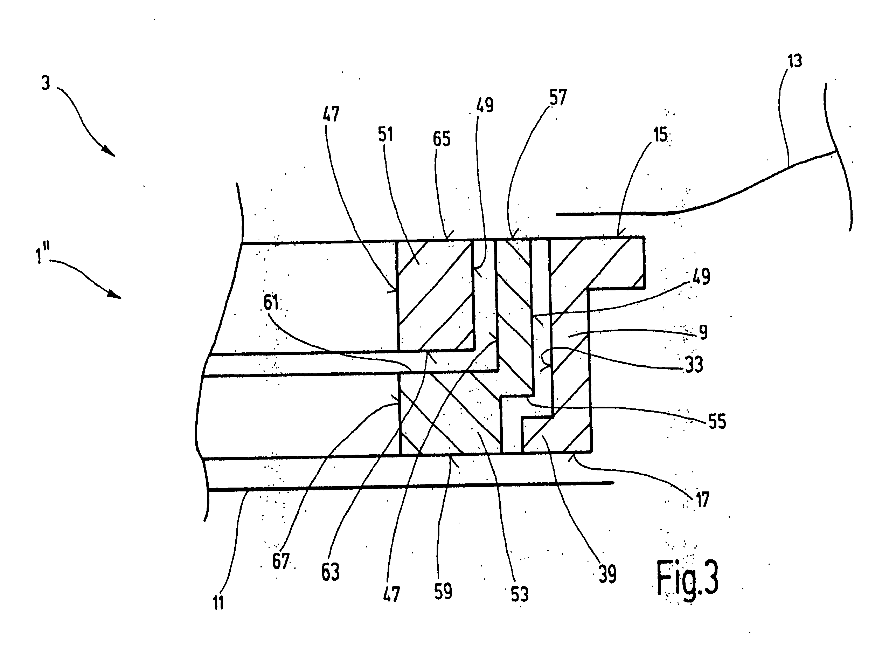

Figur 3 zeigt ein Ausführungsbeispiel eines erfindungsgemäßen Abdecksystems 1" mit zwei Adapterringen 51, 53. Gleiche Teile sind mit gleichen Bezugsziffern versehen, so dass insofern auf die Beschreibung der vorangehenden Figuren verwiesen wird.

Das Abdecksystem 1" umfasst den Abdeckring 9 und die zwei Adapterringe 51, 53, ist also an insgesamt drei verschieden große zylindrische, abzudeckende Gegenstände adaptierbar. Zu erkennen ist, dass der Anschlag des Abdeckrings 9, der durch den Vorsprung 39 gebildet wird, in zusammengefügtem Zustand mit einer im Bereich der Außenfläche 49 gebildeten, also außen liegenden Stufe 55 des Adapterrings 53 zusammenwirkt. Die Stufe 55 des Adapterrings 53 schlägt so an dem durch den Vorsprung 39 gebildeten Anschlag des Abdeckrings 9 an, dass eine obere Fläche 57 und eine untere Fläche 59 des Adapterrings 53 plan an den Verbindungsflächen 15, 17 des Abdeckrings 9 anschließen. Zum Herstellen des Presssitzes zwischen dem Abdeckring 9 und dem Adapterring 53 kann also -in Orientierung der Figur 3 gesehen- der Adapterring 53 von oben so weit in den Abdeckring 9 eingebracht werden, bis dessen Stufe 55 an dem Vorsprung 39 des Abdeckrings 9 anschlägt So ist gewährleistet, dass der Adapterring 53 richtig in dem Abdeckring 9 positioniert ist

Der Adapterring 53 weist außerdem eine im Bereich der Innenfläche 47 gebildete, also innen liegende zweite Stufe 61 auf, an der eine untere Fläche 63 des Adapterrings 51 anschlägt. Die Höhe des Adapterrings 51 ist dabei so gewählt, dass dieser sich ganz in die Stufe 61 des Adapterrings 53 einbringen lässt, also eine obere Fläche 65 des Adapterrings 51 bündig mit der oberen Fläche 57 des Adapterrings 53 und der oberen Verbindungsfläche 15 des Abdeckrings 9 abschließt

Vorteilhaft ist außerdem, dass die zweite Stufe 61 des Adapterrings 53 an eine im Wesentlichen zylindermantelförmige Innenfläche 67 angrenzt, deren Innendurchmesser dem der im Wesentlichen zylindermantelförmigen Innenfläche 47 des Adapterrings 51 entspricht. Dies ermöglicht es, wenn beide Adapterringe 51, 53 in den Abdeckring 9 eingebracht sind, diese gleichennaßen mit einem Presssitz mit einem entsprechenden Linsensystem 7 eines Mikroskops 5 zu verbinden. Das Linsensystem 7, dessen Außendurchmesser etwas größer ist als die Innendurchmesser der Flächen 47, 67 der Adapterringe 51, 53, ist in diesem Fall also mittels zwei Presssitzen so mit dem Abdecksystem 1" verbindbar, dass sich ein möglichst kleiner Abstand des Linsensystems 7 zum Sichtfenster 11 ergibt.

In einer weiteren -hier nicht dargestellten- vorteilhaften Ausgestaltung, ist an dem Adapterring 53 ein Vorsprung analog dem Vorsprung 39 des Abdeckrings 9 vorgesehen, an dem das Linsensystem 7 anschlagen kann. Dies vergrößert zwar geringfügig den minimalen Abstand des Sichtfensters 11 zum Linsensystem 7, verhindert aber dafür eine Beschädigung des Sichtfensters 11, die durch eine falsche Positionierung des Abdecksystems 1 hervorgerufen werden könnte. Dies ist insbesondere dann der Fall, wenn versehentlich das Abdecksystem 1 zu weit über das Linsensystem 7 geschoben wird. Mit herausgenommenem Adapterring 51, also wenn das Abdecksystem 1" auf einen mittleren Durchmesser adaptiert ist, erfüllt diese Funktion die Stufe 61 des Adapterrings 53.

Figur 4 zeigt ein weiteren Ausführungsbeispiel eines Abdecksystems 1"' mit einem Adapterring 35'. Gleiche Teile sind mit gleichen Bezugsziffern versehen, so dass insofern auf die Beschreibung der vorangehenden Figuren verwiesen wird.

Das Abdecksystem 1"' entspricht im Wesentlichen dem in Figur 1 dargestellten Abdecksystem 1. Der Adapterring 35' weist im Unterschied zu dem in Figur 1 dargestellten Adapterring 35 einen Vorsprung 67 auf, der eine Stufe 69 bildet, an der ein abzudeckender zylindrischer Gegenstand, also beispielsweise das Linsensystem 7, anschlagen kann. Somit ist gewährleistet, dass das Abdecksystem 1"' bei eingelegtem und auch bei herausgenommenem Adapterring 35' jeweils einen Anschlag aufweist, an dem der abzudeckende Gegenstand anschlagen kann. Somit wird in beiden Adaptionszuständen, also bei abzudeckenden Gegenständen mit kleinerem und größerem Durchmesser, gewährleistet, dass diese nicht mit dem Sichtfenster 11 in Berührung kommen und dieses etwa beschädigen.

Bei den in Figur 4 dargestellten Ausführungsbeispiel ist eine im Querschnitt U-förmige Nut 18 dargestellt, die der Aufnahme eines Sichtfensters 11 dient Denkbar ist es, hier eine randoffene Nut vorzusehen, also eine bei der der Darstellung nach Figur 4 nach unten offene Nut, in die der Sichtfenster einbringbar ist Vom Boden der Nut springen nach innen eine Anzahl von Haltenasen vor, die über das Sichtfenster greifen und dieses fixieren.

Figur 5 zeigt ein weiteres nicht zur Erfindung gehörendes Abdecksystem 1"". Gleiche Teile sind mit gleichen Bezugsziffern versehen, so dass insofern auf die Beschreibung der vorangehenden Figuren verwiesen wird.

Zu erkennen ist ein Adapterring 9' mit den Verbindungsflächen 15, 17. Über die Verbindungsflächen 15, 17 ist das Abdecksystem 1"" mit dem Sichtfenster 11 und/oder der Abdeckfolie 13, die hier der Übersichtlichkeit halber nicht dargestellt sind, verbindbar.

Der Abdeckring 9' weist zwei im Wesentlichen zylindermantelförmige Innenflächen 71, 73 mit unterschiedlichen Innendurchmessern, die durch eine Stufe 75 voneinander getrennt sind, auf. Außerdem umfasst der Abdeckring 9' eine zweite Stufe 77, die durch den Vorsprung 39 gebildet wird.

Über die im Wesentlichen zylindermantelförmigen Innenflächen 71, 73 ist der Abdeckring 9 der Vorrichtung 1"" wahlweise so über die Enden von zylindrischen Gegenständen, insbesondere das Linsensystem 7, 7', stülpbar, dass sich dort ein Presssitz ergibt Bei zylindrischen Gegenständen größeren Durchmessers ergibt sich dabei ein Presssitz zwischen der im Wesentlichen zylindermantelförmigen Innenfläche 71 und dem Gegenstand. Der Gegenstand, schlägt dabei an der Stufe 75 an. Für Gegenstände kleineren Durchmessers wird in analoger Weise der Presssitz zwischen der im Wesentlichen zylindermantelförmigen Innenfläche 73 und dem Außenumfang des Gegenstandes gebildet, wobei dieser an der Stufe 77 anschlägt Das Abdecksystem 1"", das in Verbindung mit der Abdeckfolie 13 eine Vorrichtung zum Abdecken von Mikroskopen 5 bildet, ist also ohne Entnehmen eines Adapterrings auf verschieden große zylindrische Gegenstände, insbesondere Linsensysteme 7, 7', aufsteckbar.

Anstelle des Presssitzes, der über die im Wesentlichen zylindermantelförmigen Innenflächen 31, 33; 47; 71, 73 des Abdecksystems 1, 1', 1", 1"', 1"" hergestellt wird, ist es auch denkbar, eine beliebige andere lösbare Verbindung, insbesondere eine Verklipsung, vorzusehen.

Es ist denkbar, anstelle der runden Form der Abdeckringe beziehungsweise Adapterringe auch andere Grundformen, insbesondere ovale oder polygone, vorzusehen, um das Abdecksystem auch für andere abzudeckende Gegenstände mit einer entsprechenden Außenform zu nutzen.

Schließlich ist es vorgesehen, an den im Wesentlichen zylindermantelförmigen Außen- und/oder Innenflächen der hier angesprochenen Ringe, Noppen, Rippen und/oder umlaufende Vorsprünge vorzusehen, um die Verbindung, insbesondere den Reibschluss zwischen dem zylindrischen Gegenstand und dem Abdecksystem, zu verbessern. Die Vorsprünge auf der Innenfläche der verschiedenen Ringe können eine ebene Oberfläche aufweisen, die einer gedachten Kreissekante folgt. Eine auf die Mitte einer derartigen Innenfläche eines Vorsprungs treffende Radiuslinie steht auf dieser Fläche senkrecht.

Die Größe der Noppen, Rippen und/oder Vorsprünge, also die von der zylindermandelförmigen Außen- oder Innenfläche gemessene Höhe der Noppen, Rippen und/oder Vorsprünge, richtet sich danach, wir groß die Anpresskräfte auf die benachbarten Ringe oder auf das Linsensystem sein sollen. Diese Kräfte sind auch abhängig von dem Material, aus dem die Ringe hergestellt sind.

Durch die auf der Außen- und/oder Innenfläche der hier angesprochenen Ringe Vorsprünge oder dergleichen können die Haltekräfte zwischen den verschiedenen Ringen und die auf den zylindrischen Gegenstand wirkenden Kräfte leicht auf verschiedene Anwendungsfälle eingestellt werden. Dadurch ist das Aufsetzen auf ein Linsensystem und das Herausnehmen oder Einsetzen eines Adapterrings mit definiert vorbestimmten Kräften möglich. Dabei kann im übrigen vorgesehen werden, dass auf den Ringen auf der Innen- und/oder Außenseite Noppen, Rippen und/oder Vorsprünge vorgesehen werden, um die bei der Erzeugung eines Presssitzes wirkenden Kräfte in einem weiten Rahmen einstellen zu können.

Betrachtet man die verschiedenen Ausführungsbeispiele der hier mit mindestens einem Adapterring versehenen Abdeckringe, so zeigt sich, dass die Höhe der ineinandergesetzten Ringe insbesondere dann sehr gering sein kann, wenn auf die als Anschlag dienenden Vorsprünge verzichtet wird. Dies zeigt das in Figur 3 dargestellte Ausführungsbeispiel.

Bevorzugt sind alle Ausführungsbeispiele so ausgebildet, dass deren in den Figuren nach oben gerichtete Fläche in einer Ebene liegt mit der Verbindungsfläche des Abdeckrings, was zur geringen Bauhöhe der ineinandergesetzten Ringe beiträgt Diese ist für die freie Beweglichkeit des Objektivs von besonderer Bedeutung.

Oben wurde bereits ausgeführt, dass es vorteilhaft ist, wenn der Abdeckring oder der beziehungsweise die Adapterringe einen nach innen ragenden Vorsprung aufweisen. Dieser dient dazu, dass Sichtfenster vor Beschädigungen durch ein Objektiv zu schützen. Durch diese Ausführungsform wird aber auch erreicht, dass Stöße auf das Objektiv gedämpft und damit eine Beschädigung desselben vermieden wird.

Allen anhand der Figuren 1 bis 5 erläuterten Adaptersystemen ist gemeinsam, dass es auf einfache Weise möglich ist, das Abdecksystem in Zusammenhang mit zylindrischen Gegenständen, insbesondere Linsensystemen zu verwenden, die unterschiedliche Außendurchmesser aufweisen. Dabei ist die Anpassung an verschieden Durchmesser problemlos möglich, so dass ein Austausch rasch und problemlos, insbesondere auch ohne Einsatz spezieller Werkzeuge, möglich ist.

Um die Handhabbarkeit der Adapterringe zu erleichtern, sind diese mit in den Figuren nicht dargestellten Grifflaschen versehen, die jeweils radial nach außen stehen und damit über die Außenfläche des Abdeckrings ragen. Werden mehr als ein Abdeckring verwendet, so können alle mit einer derartig radial nach außen ragenden Lasche versehen werden.

In die Oberfläche des Abdeckrings sind an die Anzahl derartiger Laschen angepasste Vertiefungen eingebracht, in die die mit den Adapterringen verbundenen Laschen im zusammengesetzten Zustand der Ringe eingesetzt werden. Die Kontur der Vertiefungen ist so an die Querschnittsform der Laschen angepasst, dass die Oberfläche der ineinandergesetzten Ringe eben ist.

Im Folgenden wird ein nicht zur Erfindung gehörigen Verfahren zum sterilen Abdecken eines Mikroskopes, insbesondere eines Operationsmikroskopes, näher erläutert. Es wird Bezug genommen auf die Figuren 1 bis 5 und die dazugehörige Beschreibung.

In einem ersten Schritt wird die Vorrichtung 3 steril aus einer Verpackung entnommen und entsprechend einer beiliegenden Gebrauchsanleitung Schritt für Schritt entfaltet.

Im darauffolgenden Schritt wird erfindungsgemäß das Abdecksystem 1, 1', 1", 1"', 1"" der Vorrichtung 3 auf den Objektivdurchmesser des abzudeckenden Mikroskops 5 adaptiert. In einem ersten Teilschritt wird dazu der Durchmesser des abzudeckenden Objektivs geprüft und mit dem Innendurchmesser des Abdecksystems 1, 1', 1", 1"', 1"" verglichen. Gegebenenfalls ist nichts weiter zu veranlassen, weil das Abdecksystem 1, 1", 1"', 1"" der Vorrichtung 3 bereits an den Objektivdurchmesser des Mikroskops 5 adaptiert ist Falls der Innendurchmesser zu klein ist, wird/werden in einem zweiten Teilschritt mittels einer Entnahmehilfe ein/mehrere Adapterring/e entnommen.

Der/die verbleibenden Adapterringe 35; 41, 43, 45; 51, 53 beziehungsweise Abdeckring 9 wird/werden im nächsten Schritt über das Objektiv, beziehungsweise das Linsensystem 7, 7', des Mikroskops 5 gestülpt und dort fixiert. Die Fixierung erfolgt bevorzugt mittels eines Presssitzes, der sich zwischen dem Objektivaußendurchmesser und der im Wesentlichen zylindermantelförmigen Innenfläche 31, 33; 47; 71, 73 der Adapterringe 35; 41, 43, 45; 51, 53 beziehungsweise des Abdeckrings 9 herstellen lässt.

Möglich ist es auch, anstelle des Presssitzes eine beliebige andere lösbare Verbindungsart vorzusehen, beispielsweise eine Verklipsung.

In einem letzten Schritt wird die Abdeckfolie 13 Ober dem Mikroskop 5 ausgebreitet und mittels Befestigungsmitteln, wie beispielsweise Bändern und/oder Klettverschlüssen an dem Mikroskop 5 fixiert. Frei bleibt lediglich das Okular des Mikroskops 5, in das der Bediener zum Beobachten des Gegenstands 23 hineinsehen kann. Bei dem Gegenstand 23 handelt es sich bevorzugt um einen steril zu haltenden Gegenstand, der -nach Durchführung des Verfahrens- durch die Vorrichtung 3 steril von dem Mikroskop 5 getrennt ist. Die Beobachtung erfolgt durch das Sichtfenster 11 hindurch.

Nach allem wird deutlich, dass das Abdecksystem problemlos an verschiedene Objektivdurchmesser eines Mikroskops anpassbar ist. Werden Abdecksysteme mit einem Abdeckring und mindestens einem Adapterring verwendet, ist es ohne weiteres möglich, den mindestens einen Adapterring auch ohne Verwendung steriler Hilfsmittel zu entfernen oder einzufügen. Die sterile Seite des Abdecksystems bleibt dabei unbeeinträchtigt, weil sie stets durch das Sichtfenster von der unsterilen Seite des Abdecksystems getrennt bleibt

## Patentansprüche

1. Abdecksystem zum Befestigen an einem vorzugsweise zylindrischen Gegenstand und/oder Abdecken eines vorzugsweise zylindrischen Gegenstands, insbesondere eines Linsensystems (7,7') eines Mikroskops (5), mit einem über ein Ende des vorzugsweise zylindrischen Gegenstands stülpbaren Abdeckring (9) und mit mindestens einem Adapterring (53), wobei der Abdeckring (9) und der Adapterring (53) jeweils eine vorzugsweise im Wesentlichen zylindermantelförmige Innenfläche (33,47) mit unterschiedlichen Durchmessern zum Adaptieren des Abdecksystems (1",1"') aufweist, wobei der mindestens eine Adapterring (53) in den über das Ende des vorzugsweise zylindrischen Gegenstands, insbesondere des Linsensysteme (7,7') des Mikroskops (5), stülpbaren Abdeckring (9) einbringbar ist, **dadurch gekennzeichnet, dass** der mindestens eine Adapterring (53) mit einer radialen nach außen stehenden und damit über die Außenfläche des Abdeckrings (9) ragenden Grifflasche versehen ist, und dass in die Oberfläche das Abdeckrings (9) an die Anzahl der Grifflaschen angepasste Vertiefungen eingebracht sind, in die die mit dem mindestens einem Adapterring verbundene Grifflasche im zusammengesetzten Zustand der Ringe des Abdecksystems eingesetzt werden, wobei die Kontur der Vertiefung so an die Querschnittsform der Grifflaschen angepasst ist, dass die Oberfläche der ineinander gesetzten Ringe des Abdecksystems eben ist, dass der mindestens eine Adapterring (53) einen Anschlag, insbesondere eine Stufe (61), für einen weiteren Adapterring (51) aufweist, und dass auf der Innen- und/oder Außenfläche des Abdeckrings und/oder der Adapterringe Noppen, Rippen und/oder Vorsprünge vorgesehen sind.

2. Abdecksystem nach Anspruch 1, **gekennzeichnet durch** mindestens einen Adapterring (35:35':41,43,45;51,53).

3. Abdecksystem nach Anspruch 1 oder 2, **dadurch gekennzeichnet, dass** der mindestens eine Adapterring (35;35';41,43,45;51,53) in den über das Ende des zylindrischen Gegenstandes, insbesondere des Linsensystems (7,7') des Mikroskops (5), stülpbaren Abdeckring (9) einbringbar ist.

4. Abdecksystem nach einem der vorhergehenden Ansprüche, **dadurch gekennzeichnet, dass** der mindestens eine Adapterring (35;35';41,43,45;51,53) und der Abdeckring (9) jeweils eine der im wesentlichen zylindermantelförmigen Innenflächen (31,33; 47) aufweist.

5. Abdecksystem nach einem der vorhergehenden Ansprüche, **dadurch gekennzeichnet, dass** der Adapterring (35;35';41,43, 45;51,53) und der Abdeckring (9) einen Presssitz bilden.

6. Abdecksystem nach einem der vorhergehenden Ansprüche, **dadurch gekennzeichnet, dass** der Abdeckring (9) einen Anschlag, insbesondere einen Vorsprung (39), für den mindestens einen Adapterring (35;35';41,43,45;51,53) aufweist.

7. Abdecksystem nach einem der vorhergehenden Ansprüche, **dadurch gekennzeichnet, dass** der mindestens eine Adapterring (35;35';41,43,45;51,53) über das Ende eines zylindrischen Gegenstandes kleineren Durchmessers stülpbar ist.

8. Abdecksystem nach einem der vorhergehenden Ansprüche, **dadurch gekennzeichnet, dass** der Abdeckring (9) und drei weitere Adapterringe (41,43,45) vorgesehen sind, wobei diese jeweils ineinander einbringbar sind.

9. Abdecksystem nach einem der vorhergehenden Ansprüche, **dadurch gekennzeichnet dass** das Abdecksystem (1) durch Herausnehmen oder Einbringen des mindestens einen Adapterrings (35;35';41,43,45;51,53) an verschieden große Gegenstände adaptierbar ist.

10. Abdecksystem nach einem der vorhergehenden Ansprüche, **gekennzeichnet durch** ein Sichtfenster (11).

11. Abdecksystem nach einem der vorhergehenden Ansprüche, **dadurch gekennzeichnet dass** das Sichtfenster (11) Glas und/oder Plexiglas und/oder eine bevorzugt 300 µm starke Polycarbonatfolie und/oder Cycloolefincopolymer, insbesondere 300 µm stark, aufweist.

12. Abdecksystem nach einem der vorhergehenden Ansprüche, **dadurch gekennzeichnet, dass** das Sichtfenster (11) mit dem Abdeckring (9;9') verbunden ist.

13. Abdecksystem nach einem der vorhergehenden Ansprüche, **dadurch gekennzeichnet, dass** das Sichtfenster (11) eine Vorrichtung (19) zum Trennen desselben vom Abdeckring (9;9') aufweist.

14. Abdecksystem nach einem der vorhergehenden Ansprüche, **dadurch gekennzeichnet, dass** der Abdeckring (9) sowie der mindestens eine Adapterring (35;35';41,43,45;51,53) aus einem anpassungsfähigen, vorzugsweise elastisch verformbaren, Material, bevorzugt aus welchem Polyethylen, besteht.

15. Abdecksystem nach einem der vorhergehenden Ansprüche, **dadurch gekennzeichnet, dass** der mindestens eine Adapterring (35;35';41,43,45;51,53) und/oder der Abdeckring (9) einen U-förmigen Querschnitt aufweist.

16. Abdecksystem nach einem der vorhergehenden Ansprüche, **dadurch gekennzeichnet, dass** der Abdeckring (9) eine Verbindungsfläche (15) zum Verbinden mit einer Abdeckfolie (13) und/oder eine Verbindungsfläche (17) oder eine, insbesondere zumindest teilweise, umlaufende Nut zum Verbinden mit dem Sichtfenster (11) aufweist.

17. Abdecksystem nach Anspruch 1, **dadurch gekennzeichnet, dass** der Abdeckring (9') mindestens zwei im wesentlichen zylindermantelförmigen Innenflächen (71,73) aufweist.

18. Vorrichtung zum sterilen Abdecken eines Mikroskops, insbesondere eines Operationsmikroskops, mit einer das Mikroskop einhüllenden Abdeckfolie, **gekennzeichnet durch** ein Abdecksystem (1";1"') nach einem der Ansprüche 1 bis 19.

## Claims

1. A covering system to be attached to a preferably cylindrical object and/or covering a preferably cylindrical object, particularly a lens system (7, 7') of a microscope (5), comprising a covering ring (9) that can be put over one end of the preferably cylindrical object and comprising at least one adapter ring (53), wherein said covering ring (9) and said adapter ring (53) each comprise an inner surface (33; 47) that has preferably and essentially the shape of a cylinder jacket and has different diameters allowing adaptation of the covering system (1", 1"'), wherein the at least one adapter ring (53) can be inserted in the covering ring (9) that can be put over the end of the preferably cylindrical object, particularly the lens system (7, 7') of the microscope (5), **characterized in that** the at least one adapter ring (53) is provided with a gripping strap projecting in a radially outward direction and, thus, projecting beyond the outer surface of the covering ring (9) and that recesses are provided in the surface of the covering ring (9) that are matching the number of the gripping straps, wherein the gripping strap that is connected to the at least one adapter ring are inserted in said recesses while the rings are in their joined condition, wherein the contour of the recess is adjusted to the cross-sectional shape of the gripping straps such that the surface of the rings of the covering system that are inserted in each other is plane, that the at least one adapter ring (53) comprises a stop, particularly a step (61) for a further adapter ring (51), and that knobs, ribs and/or projections are provided on the inner and/or outer surface of the covering ring and/or the adapter rings.

2. A covering system according to Claim 1, **characterized in that** it comprises at least one adapter ring (35; 35'; 41, 43, 45; 51, 53).

3. A covering system according to Claim 1 or 2, **characterized in that** the at least one adapter ring (35; 35'; 41, 43, 45; 51, 53) can be inserted in the covering ring (9) that can be put over the end of the cylindrical object, particularly the lens system (7, 7') of the microscope (5).

4. A covering system according to anyone of the preceding claims, **characterized in that** the at least one adapter ring (35; 35'; 41, 43, 45; 51, 53) and the covering ring (9) each comprise one of the inner surfaces (31, 33; 47) essentially having the shape of a cylinder jacket.

5. A covering system according to anyone of the preceding claims, **characterized in that** the adapter ring (35; 35'; 41, 43, 45; 51, 53) and the covering ring (9) form a press fit.

6. A covering system according to anyone of the preceding claims, **characterized in that** the covering ring (9) comprises a stop, particularly a projection (39), for the at least one adapter ring (35; 35'; 41, 43, 45; 51, 53).

7. A covering system according to anyone of the preceding claims, **characterized in that** the at least one adapter ring (35; 35'; 41, 43, 45; 51, 53) can be put over the end of a cylindrical object having a smaller diameter.

8. A covering system according to anyone of the preceding claims, **characterized in that** the covering ring (9) and three further adapter rings (41, 43, 45) are provided, wherein these rings can each be inserted in each other.

9. A covering system according to anyone of the preceding claims, **characterized in that** the covering system (1) can be adjusted to objects of varying size by removing or inserting the at least one adapter ring (35; 35'; 41, 43, 45; 51, 53).

10. A covering system according to anyone of the preceding claims, **characterized by** an inspection window (11).

11. A covering system according to anyone of the preceding claims, **characterized in that** the inspection window (11) comprises glass and/or plexiglass and/or a polycarbonate foil that is preferably 300 µm thick and/or cyclo-olefine copolymer that is particularly 300 µm thick.

12. A covering system according to anyone of the preceding claims, **characterized in that** the inspection window (11) is connected to the covering ring (9; 9').

13. A covering system according to anyone of the preceding claims, **characterized in that** the inspection window (11) comprises a device (19) allowing to remove it from the covering ring (9; 9').

14. A covering system according to anyone of the preceding claims, **characterized in that** the covering ring (9) as well as the at least one adapter ring (35; 35'; 41, 43, 45; 51, 53) consists of an adaptable, preferably elastically formable material, preferably of soft polyethylene.

15. A covering system according to anyone of the preceding claims, **characterized in that** the at least one adapter ring (35; 35'; 41, 43, 45; 51, 53) and/or the covering ring (9) comprises a U-shaped cross-section.

16. A covering system according to anyone of the preceding claims, **characterized in that** the covering ring (9) comprises a connection area (15) to be connected to a covering foil (13) and/or a connection area (17) or a groove to be connected to the inspection window (11), said groove being, particularly at least in part, circumferential.

17. A covering system according to Claim 1, **characterized in that** the covering ring (9') comprises at least two inner surfaces (71, 73) essentially having the shape of a cylinder jacket.

18. A device for covering a microscope in a sterile manner, in particular an operating microscope, comprising a covering foil enveloping the microscope, **characterized by** a covering system (1 ", 1"') according to anyone of Claims 1 to 17.

## Revendications

1. Système de revêtement permettant de fixer à un objet de préférence cylindrique et / ou revêtement d'un objet de préférence cylindrique, notamment d'un système de lentilles (7, 7') d'un microscope (5), comprenant un anneau de revêtement (9) pouvant être retourné sur une extrémité de l'objet de préférence cylindrique et au moins un anneau d'adaptateur (53), l'anneau de revêtement (9) et l'anneau de l'adaptateur (53) présentant l'un et l'autre, de préférence principalement une surface interne en forme de revêtement cylindrique (33, 47) ayant des diamètres différents permettant l'adaptation du système de revêtement (1", 1"', le au moins un anneau d'adaptateur (53) pouvant être mis en place dans l'anneau de revêtement (9) pouvant être retourné sur l'extrémité de l'objet de préférence cylindrique, notamment du système de lentilles (7, 7') du microscope (5), **caractérisé en ce que** le au moins un anneau d'adaptateur (53) est pourvu d'une dragonne orientée de façon radiale vers l'extérieur et s'élevant par conséquent au-dessus de la surface externe de l'anneau de revêtement (9) et **en ce que** des cavités adaptées au nombre de dragonnes sont insérées sur la surface de l'anneau de revêtement (9), dans lesquelles cavités la dragonne liée au, au moins un, anneau d'adaptateur sont insérés dans l'état composé des anneaux du système de revêtement, le contour de la cavité étant adapté à la forme d'une section transversale des dragonnes de telle sorte que la surface des anneaux du système de revêtement positionnés les uns dans les autres est plane, le au moins un anneau d'adaptateur (53) présente un taquet, notamment un niveau (61) pour un autre anneau d'adaptateur (51) et **en ce que** des boutons, des nervures et / ou de parties saillantes sont prévus sur la surface interne et / ou externe de l'anneau de revêtement et / ou des anneaux de revêtement.

2. Système de revêtement selon la revendication 1, **caractérisé par** au moins un anneau d'adaptateur (35, 35'; 41, 43, 45 ; 51, 53).

3. Système de revêtement selon la revendication 1 ou 2, **caractérisé en ce que** le au moins un anneau d'adaptateur (35, 35'; 41, 43, 45 ; 51, 53) peut être mis en place dans l'anneau de revêtement (9) pouvant être retourné sur l'extrémité de l'objet cylindrique, notamment du système de lentilles (7, 7') du microscope (5).

4. Système de revêtement selon l'une quelconque des revendications précédentes, **caractérisé en ce que** le au moins un anneau d'adaptateur (35, 35'; 41, 43, 45 ; 51, 53) et l'anneau de revêtement (9) présentent l'un et l'autre principalement une des surfaces internes en forme de revêtement cylindrique (31, 33 ; 47).

5. Système de revêtement selon l'une quelconque des revendications précédentes, **caractérisé en ce que** l'anneau d'adaptateur (35, 35'; 41, 43, 45 ; 51, 53) et l'anneau de revêtement (9) forment un ajustage serré.

6. Système de revêtement selon l'une quelconque des revendications précédentes, **caractérisé en ce que** l'anneau de revêtement (9) présente un taquet, notamment une partie saillante (39) pour le au moins un anneau d'adaptateur (35, 35'; 41, 43, 45 ; 51, 53).

7. Système de revêtement selon l'une quelconque des revendications précédentes, **caractérisé en ce que** le au moins un anneau d'adaptateur (35, 35'; 41, 43, 45 ; 51, 53) peut être retourné sur l'extrémité d'un objet cylindrique de moindre diamètre.

8. Système de revêtement selon l'une quelconque des revendications précédentes, **caractérisé en ce que** l'anneau de revêtement (9) et trois autres anneaux d'adaptateurs (41, 43, 45) sont prévus, ceux-ci étant à chaque fois insérés chacun les uns dans les autres.

9. Système de revêtement selon l'une quelconque des revendications précédentes, **caractérisé en ce que** le système de revêtement (1) peut être adapté par retrait ou insertion du au moins un anneau d'adaptateur (35, 35'; 41, 43, 45 ; 51, 53), à différents objets de taille importante.

10. Système de revêtement selon l'une quelconque des revendications précédentes, **caractérisé en ce qu'**il dispose d'un voyant (11).

11. Système de revêtement selon l'une quelconque des revendications précédentes, **caractérisé en ce que** le voyant (11) présente un verre et / ou un plexiglas et / ou une feuille de polycarbonate épaisse de de préférence 300 µm et / ou un copolymère de cyclooléfine, notamment épais de 300 µm.

12. Système de revêtement selon l'une quelconque des revendications précédentes, **caractérisé en ce que** le voyant (11) est liée à l'anneau de revêtement (9, 9').

13. Système de revêtement selon l'une quelconque des revendications précédentes, **caractérisé en ce que** le voyant (11) présente un dispositif (19) pour la séparer de l'anneau de revêtement (9, 9').

14. Système de revêtement selon l'une quelconque des revendications précédentes, **caractérisé en ce que** l'anneau de revêtement (9) ainsi que le au moins un anneau d'adaptateur (35, 35'; 41, 43, 45 ; 51, 53) se compose d'un matériau adaptable, de préférence déformable élastiquement, de préférence en polyéthylène mou.

15. Système de revêtement selon l'une quelconque des revendications précédentes, **caractérisé en ce que** le au moins un anneau d'adaptateur (35, 35'; 41, 43, 45 ; 51, 53) et/ou l'anneau de revêtement (9) présente d'une section transversale en forme de U.

16. Système de revêtement selon l'une quelconque des revendications précédentes, **caractérisé en ce que** l'anneau de revêtement (9) présente une surface de liaison (15) pour la liaison avec une feuille de revêtement (13) et / ou une surface de liaison (17) ou une rainure périphérique ou au moins partiellement périphérique en vue d'une liaison avec le voyant (11).

17. Système de revêtement selon la revendication 1, **caractérisé en ce que** l'anneau de revêtement (9') présente au moins deux surfaces internes (71, 73) sous forme de revêtement principalement cylindrique.

18. Dispositif pour le revêtement stérile d'un microscope, notamment d'une microscope opératoire, présentant une feuille de revêtement enveloppant le microscope, **caractérisé par** un système de revêtement (1" ; 1"') selon l'une quelconque des revendications 1 à 19.
